# EUROPEAN PATENT APPLICATION

(11) **EP 4 026 481 A1**
(43) Date of publication of application: **13.07.2022**
(21) Application number: 19944246.8
(22) Date of filing: 05.09.2019
(51) Int. Cl.: A61B 5/00, A61B 5/16, G16H 50/20, C12Q 1/68

(54) **SYSTEM PROVIDING SOLUTION THROUGH INTEGRATION OF DNA APTITUDE TEST AND PERSONALITY/APTITUDE TEST**

(30) Priority: 02.09.2019 KR 20190108356
(71) Applicant: Clinomics Inc., Ulsan 44919 (KR)
(72) Inventor: CHO, Yun Sung, Yongin-si Gyeonggi-do 16953 (KR); CHO, Su An, Hwaseong-si Gyeonggi-do 18320 (KR); KIM, Byung Chul, Suwon-si Gyeonggi-do 16713 (KR); BHAK, Jong Hwa, Ulju-gun Ulsan 44936 (KR)
(74) Representative: Frenkel, Matthias Alexander
(86) International application number: PCT/KR2019/011485
(87) International publication number: WO 2021/045269

(57) **Abstract**

The present invention relates to a system providing a solution through integration of a DNA aptitude test and a personality/aptitude test. The technical problem to be solved is to enhance the personalization, precision, efficiency, and effectiveness of personality and aptitude tests through tests of a person's genetic characteristics, inclination, and aptitude together with a personality/aptitude test. According to an embodiment, a system providing a solution through integration of a DNA aptitude test and a personality/aptitude test is disclosed, the system comprising: a personality/aptitude test information providing unit for providing personality/aptitude test information through a personality/aptitude test of a subject; a DNA aptitude test information providing unit for providing DNA aptitude test information by analyzing the genetic inclination of the subject on the basis of genetic test results of the subject; and a solution providing unit which analyzes genetic inclination and environmental factors, which have caused results of the personality/aptitude test information, by comparing the personality/aptitude test information with the DNA aptitude test information, and provides a solution needed for the career and education of the subject on the basis of the corresponding analysis result, the DNA aptitude test information, and the personality/aptitude test information.

## Description

### TECHNICAL FIELD

Embodiments of the present invention relate to a solution providing system through fusion of a DNA aptitude test and an aptitude/personality test.

### BACKGROUND ART

There are various personality/aptitude test methods to identify personality or aptitude in the education of children and adolescents or the re-education of adults.

Most of the conventional personality/aptitude test methods are performed on a prepared questionnaire for a certain period of time.

The result of this personality/aptitude test scheme is highly likely to change depending on the psychological environment in answering given questions for a limited period of time, and so is it depending on the testee's will, level of knowledge, temporal environment and spatial environment, and emotions. Further, since the test results are quantified or categorized and interpreted, the conventional methods do not reflect individuals' specificity.

Further, there is a limit in figuring out the characteristics of behavior, emotion, recognition, judgment, etc., which are unconsciously performed.

Therefore, a need arises for developing an aptitude test system and method that may more accurately predict an individual's talent by using innate characteristics to address the foregoing issues. However, there is no or little research on analyzing individual genetic information and gathering aptitude information gathered therefrom.

### DETAILED DESCRIPTION OF THE INVENTION

### TECHNICAL PROBLEMS

According to an embodiment of the present invention, there is provided a system for providing a solution through fusion of a DNA aptitude test and a personality/aptitude test which may increase the customization, precision, efficiency and effectiveness of personality and aptitude tests through individual genetic characteristics, predisposition, and aptitude tests in addition to personality/aptitude tests.

### MEANS TO ADDRESS THE PROBLEMS

According to an embodiment of the present invention, a system for providing a solution through fusion of a DNA aptitude test and a personality/aptitude test comprises a personality/aptitude test information providing unit providing personality/aptitude test information through a personality/aptitude test on a testee; a DNA aptitude test information providing unit analyzing the testee's genetic predisposition based on a result of a genetic test on the testee and providing DNA aptitude test information; and a solution providing unit comparing the personality/aptitude test information with the DNA aptitude test information, analyzing a genetic predisposition and an environmental factor resulting from a result of the personality/aptitude test information, and providing a solution necessary for a career path and education of the testee based on a result of the analysis and the DNA aptitude test information and the personality/aptitude test information.

Further, the personality/aptitude test information providing unit may perform the personality/aptitude test on the testee through at least one scheme of an online scheme and an offline scheme and provide the result of the personality/aptitude test on the testee.

Further, the DNA aptitude test information providing unit may include a genetic predisposition information providing unit determining the testee's genotype using a DNA marker having a specific genetic predisposition and providing genetic predisposition information for the testee according to a result of the determination; and a genetic aptitude information providing unit providing a score for the genetic aptitude information for the testee based on the genetic predisposition information provided through the genetic predisposition providing unit and a pre-established genetic aptitude information database.

Further, the solution providing unit may include a first solution providing unit comparing the personality/aptitude test information and the DNA aptitude test information for the testee, if the personality/aptitude test information is not different from the DNA aptitude test information, analyzing whether the result of the personality/aptitude test information comes from the genetic predisposition or the environmental factor, if the result of the personality/aptitude test information is analyzed as coming from the genetic predisposition, providing an environmental solution for enhancing the testee's personality/aptitude by enhancing the environmental factor, and if the result of the personality/aptitude test information is analyzed as coming from the environmental factor, providing a genetic solution for enhancing the testee's personality/aptitude by enhancing the genetic predisposition; and a second solution providing unit comparing the personality/aptitude test information and the DNA aptitude test information for the testee and, if the personality/aptitude test information is determined to be different from the DNA aptitude test information, providing a solution for enhancing the testee's personality/aptitude based on the personality/aptitude test information.

Further, the system may further comprise a personality/aptitude variation trend information providing unit creating a database for the personality/aptitude test information through solution providing unit, the DNA aptitude test information, and a result of analysis on solution information and providing information about a personality/aptitude variation trend when the testee's personality/aptitude is retested.

Further, the solution providing unit may further include a first messenger operating unit measuring a similarity between the personality/aptitude test information and the DNA aptitude test information for testees, extracting testees having a preset reference similarity or more, opening a chat room for conversation between the extracted testees, matching the testees, and operating counseling and education.

Further, the solution providing unit may further include a second messenger operating unit setting testees having a difference between the personality/aptitude test information and the DNA aptitude test information as subjects, extracting testees among the subjects for which the personality/aptitude test information and the DNA aptitude test information are complementarily different from each other, opening a chat room for conversation between the extracted testees, matching the testees, and operating counseling and education.

Further, the personality/aptitude test may include at least one test item of intelligence, mathematical computational ability, positive thinking, sleep pattern, subjective euphoria, life satisfaction, neuroticism, extroverted personality, education level, information processing speed, mathematical ability, verbal memory, ability of learning from failure, altruism, athletic ability, muscle composition, injury risk, core body temperature stability during exercise, lactate threshold reaction for aerobic exercise, and exercise heart rate for regular exercise training.

### EFFECTS OF THE INVENTION

According to the present invention, it is possible to provide a system for providing a solution through fusion of a DNA aptitude test and a personality/aptitude test which may increase the customization, precision, efficiency and effectiveness of personality and aptitude tests through individual genetic characteristics, predisposition, and aptitude tests in addition to personality/aptitude tests.

### BRIEF DESCRIPTION OF THE DRAWINGS

FIG. 1 is a schematic view illustrating an overall configuration of a solution provision system through fusion of a DNA aptitude test and a personality/aptitude test according to an embodiment of the disclosure.
FIG. 2 is a block diagram illustrating an overall configuration of a solution provision system through fusion of a DNA aptitude test and a personality/aptitude test according to an embodiment of the disclosure.
FIG. 3 is a block diagram illustrating a configuration of a DNA aptitude test information providing unit according to an embodiment of the disclosure.
FIG. 4 is a block diagram illustrating a configuration of a solution providing unit according to an embodiment of the disclosure.
FIG. 5 is a view illustrating a solution providing scheme of a first solution providing unit according to an embodiment of the disclosure.
FIG. 6 is a view illustrating a solution providing scheme of a second solution providing unit according to an embodiment of the disclosure.
FIG. 7 is a view illustrating a chat room opening scheme of a first messenger operating unit according to the present invention.
FIG. 8 is a view illustrating a chat room opening scheme of a second messenger operating unit according to the present invention.

### BEST MODE TO PRACTICE THE INVENTION

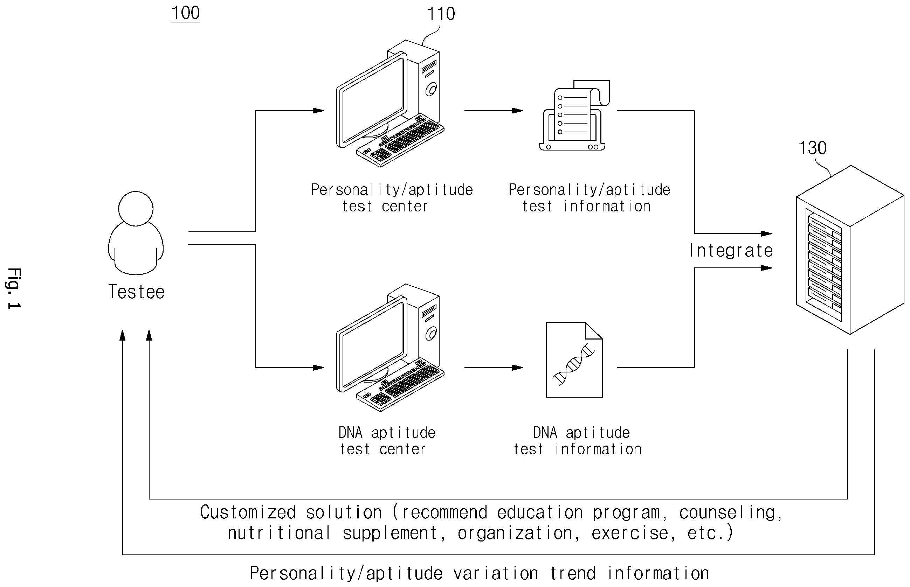

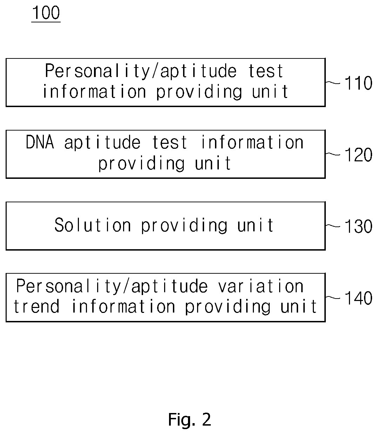

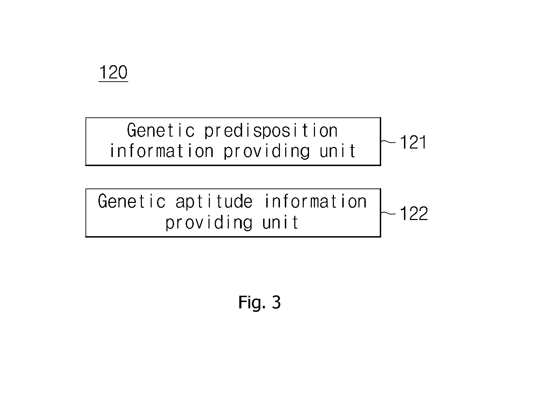

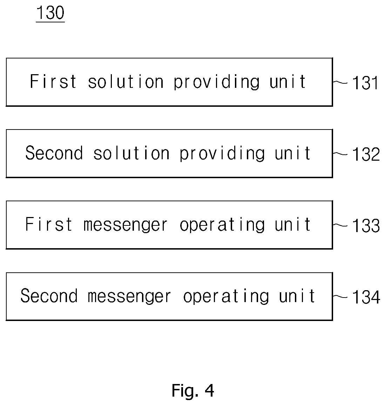

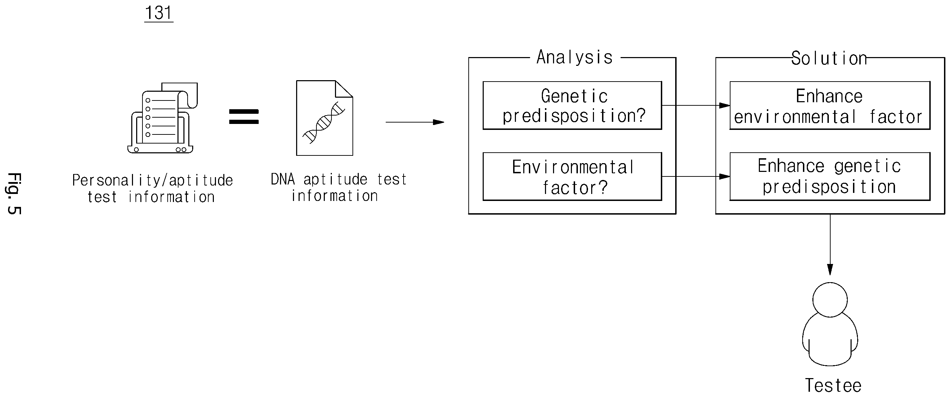

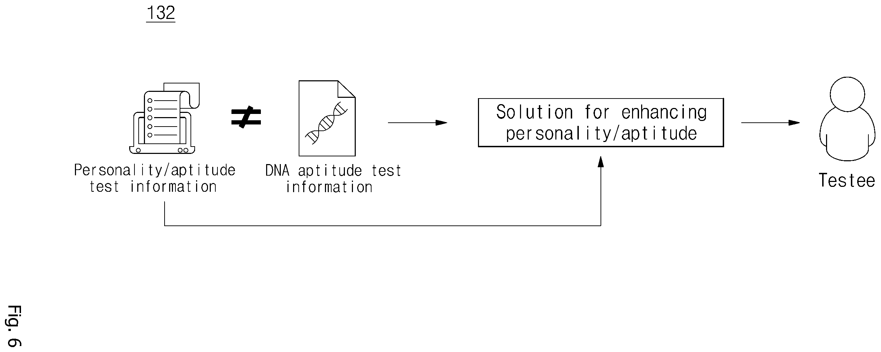

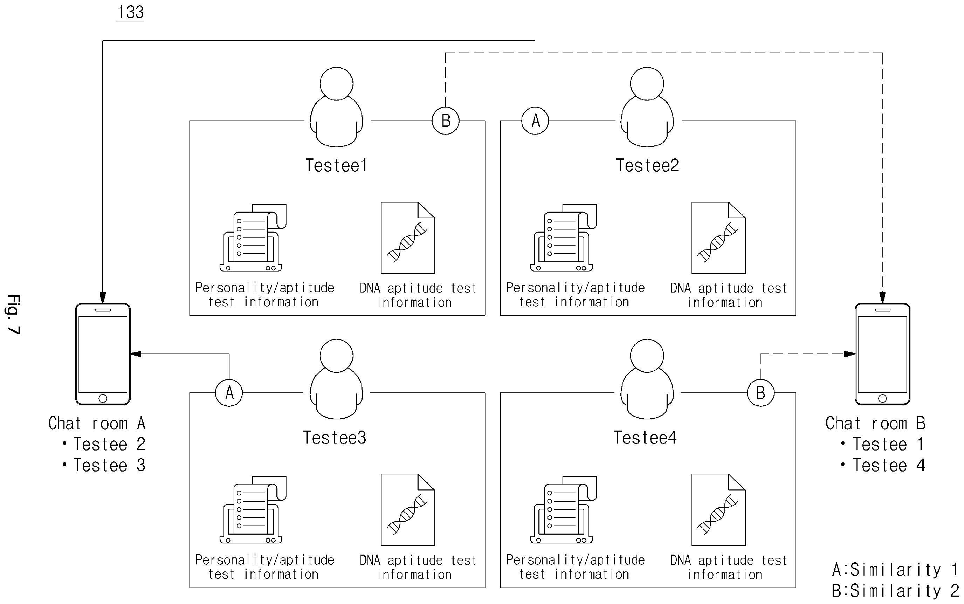

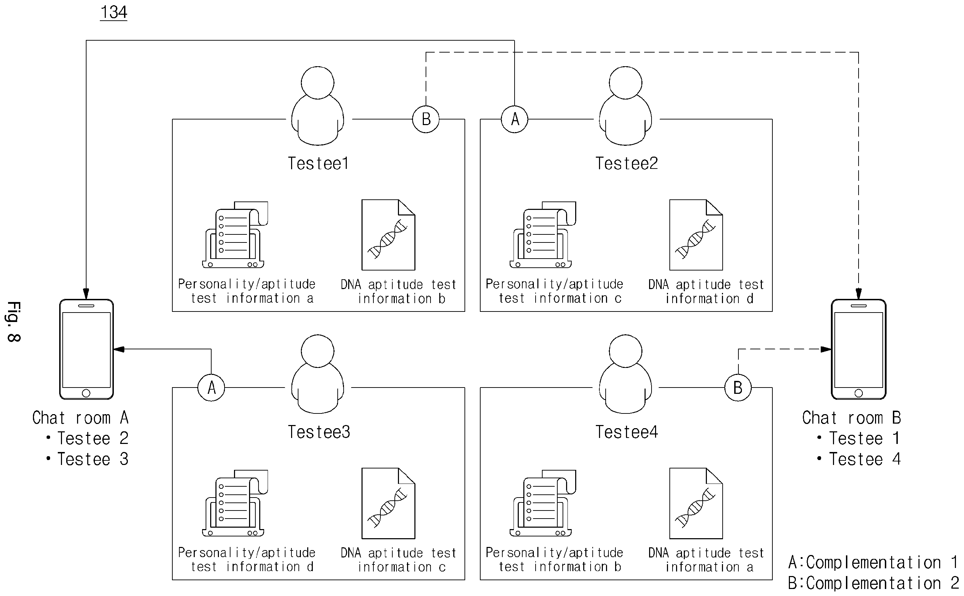

FIG. 1 is a schematic view illustrating an overall configuration of a solution provision system through fusion of a DNA aptitude test and a personality/aptitude test according to an embodiment of the disclosure. FIG. 2 is a block diagram illustrating an overall configuration of a solution provision system through fusion of a DNA aptitude test and a personality/aptitude test according to an embodiment of the disclosure. FIG. 3 is a block diagram illustrating a configuration of a DNA aptitude test information providing unit according to an embodiment of the disclosure. FIG. 4 is a block diagram illustrating a configuration of a solution providing unit according to an embodiment of the disclosure. FIG. 5 is a view illustrating a solution providing scheme of a first solution providing unit according to an embodiment of the disclosure. FIG. 6 is a view illustrating a solution providing scheme of a second solution providing unit according to an embodiment of the disclosure. FIG. 7 is a view illustrating a chat room opening scheme of a first messenger operating unit according to the present invention. FIG. 8 is a view illustrating a chat room opening scheme of a second messenger operating unit according to the present invention.

Referring to FIGS. 1 and 2, according to an embodiment of the disclosure, a solution provision system 100 through fusion of a DNA aptitude test and a personality/aptitude test includes a personality/aptitude test information providing unit 110, a DNA aptitude test information providing unit 120, a solution providing unit 130, and a personality/aptitude variation trend information providing unit 140.

The personality/aptitude test information providing unit 110 may provide personality/aptitude test information through a personality/aptitude test on a testee. To this end, the personality/aptitude test information providing unit 110 may provide personality/aptitude test results for each testee by performing a personality/aptitude test on each testee through at least one scheme among online and offline schemes. For example, the personality/aptitude test may be performed through an online survey scheme through a mobile application, mobile web, internet web, etc., or the testee may visit a personality/aptitude test center and perform a personality/aptitude test by filling out the questionnaire. However, for more diverse personality/aptitude tests, the personality/aptitude test is not limited to the conventional survey scheme, but it may be performed together with medical tests, physical tests, etc.

For example, the personality/aptitude test may include at least one test item of intelligence, mathematical computational ability, positive thinking, sleep pattern, subjective euphoria, life satisfaction, neuroticism, extroverted personality, education level, information processing speed, mathematical ability, verbal memory, ability of learning from failure, altruism, athletic ability, muscle composition, injury risk, core body temperature stability during exercise, lactate threshold reaction for aerobic exercise, and exercise heart rate for regular exercise training, but in this embodiment, examples of the personality/aptitude test are not limited thereto but may rather include more various test items. If the testee has a desired career path or education (including re-education) or if the testee wants to get a test for items related to the career path, education, marriage, etc. it is preferable to perform a customized personality/aptitude test considering such items. Of course, testee may sign up for membership, including registering various personal information, before receiving the personality/aptitude test.

The DNA aptitude test information providing unit 120 may analyze the testee's genetic predisposition based on the testee's genetic test result and provide DNA aptitude test information. In order for the DNA aptitude test information providing unit 120 to obtain DNA aptitude test information, the testee needs to visit a DNA aptitude test center, and the testee's DNA samples, such as saliva, spheroidal epithelial cells, and blood, need to be collected. If the testee wants to get test for some items, such as a specific career path, education, or marriage, it is preferable to conduct the DNA aptitude test considering such items. Of course, testee may sign up for membership, including registering various personal information, before receiving the DNA aptitude test.

To this end, the DNA aptitude test information providing unit 120 may include a genetic predisposition information providing unit 121 and a genetic aptitude information providing unit 122 as illustrated in FIG. 3.

The genetic predisposition information providing unit 121 may determine the genotype of the testee by a DNA marker having a specific genetic predisposition and may provide genetic predisposition information for each testee according to the result of determination. The DNA marker means to mark an area of DNA and functions to easily distinguish it from other DNA. Through the DNA marker, a specific genetic predisposition may be distinguished or determined for each testee. To this end, the genotype of the DNA marker, which is a genetic marker of the testee, may be typed, and the genetic predisposition (personality, physical constitution, thinking ability, etc.) of the testee may be determined according to the result of typing, that is, the genotype.

The genetic aptitude information providing unit 122 may derive genetic aptitude information for each testee based on the genetic predisposition information provided through the genetic predisposition providing unit 121 and a pre-established genetic aptitude information database and may be scored and provided.

The solution providing unit 130 may compare the personality/aptitude test information received from the personality/aptitude test information providing unit 110 with the DNA aptitude test information and analyze factors originating from the result of the personality/aptitude test information. More specifically, the genetic predisposition and environmental factors resulting from the personality/aptitude test information may be analyzed. It is possible to provide various customized solutions necessary for the testee's career path and education based on the result of analysis, DNA aptitude test information, and personality/aptitude test information.

To this end, as illustrated in FIG. 4, the solution providing unit 130 may include a first solution providing unit 131, a second solution providing unit 132, a first messenger operating unit 133, and a second messenger operating unit 134.

The first solution providing unit 131 may compare the personality/aptitude test information for the testee with the DNA aptitude test information and, if it is determined that the personality/aptitude test information is not different from the DNA aptitude test information, analyze whether the result of the personality/aptitude test comes from genetic predisposition or environmental factors. As a result, if it is analyzed that the result of personality/aptitude test information comes from the diagnostic predisposition, the first solution providing unit 131 may provide an environmental solution for enhancing the testee's personality/aptitude by enhancing the environmental factors. If it is analyzed that the result of personality/aptitude test information comes from the environmental factors, the first solution providing unit 131 may provide a genetic solution for enhancing the testee's personality/aptitude by enhancing the genetic predisposition.

For example, if the personality/aptitude test information and the DNA aptitude test information for the testee are obtained as substantially the same results as illustrated in FIG. 5, the first solution providing unit 131 may analyze whether the result of the personality/aptitude test information about the testee is due to genetic predisposition or environmental factors. For example, if the testee's career path is related to 'athlete', and both the personality/aptitude test information and the DNA aptitude test information show positive results in relation to 'athlete', it may be analyzed whether there is an environmental factor around the testee, resulting in the personality/aptitude test result of 'athlete' and, if any, how much it affects, or grasp whether the DNA aptitude test result for the testee and people close to the testee (e.g., parents, brothers, sisters, etc.) have a factor related to 'athlete' and, if any, how much it affects.

If a result due to genetic predisposition is derived from this analysis result, the first solution providing unit 131 may provide a solution for enhancing environmental factors. For example, if the result of the personality/aptitude test, 'athlete,' is analyzed as coming from genetic predisposition, the first solution providing unit 131 may provide a solution for enhancing the environmental factors so that the testee's personality/aptitude, as an athlete, may be further enhanced. Further, the first solution providing unit 131 may recommend customized exercise related to athletic ability, recovery ability, aerobic ability, muscle composition, injury risk, etc., as well as psychological counseling, educational programs (kindergarten, schools, private academy, homeschooling workbooks, etc.), educational institutions, etc. considering the genetic or genotype analysis results for the testee.

Further, if the analysis result comes from environmental factors, the first solution providing unit 131 may provide a solution for enhancing genetic predisposition. For example, if it is analyzed that the result of the personality/aptitude test, i.e., 'athlete,' comes from environmental factors, the first solution providing unit 131 may provide a solution for enhancing genetic predisposition to further enhance the testee's personality/aptitude as athlete and may recommend customized nutritional supplements, medical technology, etc., considering the testee's genetic or genotype analysis results as the solution.

The second solution providing unit 132 may compare the personality/aptitude test information for the testee with the DNA aptitude test information and, if it is determined that the personality/aptitude test information is different from the DNA aptitude test information, provide a solution for enhancing the testee's personality/aptitude based on the personality/aptitude test information.

For example, if the personality/aptitude test information shows positive results in relation to 'athlete', and the DNA aptitude test information shows positive results in relation to 'literature,' so that the difference between them is clear as illustrated in FIG. 6, the second solution providing unit 132 may recommend psychological counseling and educational programs (kindergarten, school, private academy, homeschooling workbooks, etc.), the training period, etc. to enhance the testee's personality/aptitude based on the personality/aptitude test information related to 'athlete'.

The first messenger operating unit 133 may measure the similarity between personality/aptitude test information and DNA aptitude test information for testees, extract testees for whom the similarity is a preset value or more, and open a chat room for the extracted testees, match testees and operate consultation.

For example, as illustrated in FIG. 7, personality/aptitude test information and DNA aptitude test information for each of testees 1, 2, 3, and 4 are stored in a database. Then, for each testee, the personality/aptitude test information and the DNA aptitude test information are compared between each other, and similarity is analyzed based on a specific criterion. Testees for whom the similarity is a preset value or more are classified. For example, for similarity A, testee 2 and testee 3 may be classified into one group and, for similarity B, testee 1 and testee 4 may be classified into one group. For each group, a chat room is opened with the subject of the corresponding personality/aptitude test information and DNA aptitude test information, and a chance of introduction may be provided. As necessary, a counselor may intervene to proceed with counselling with the testees. Such a solution may be provided.

The second messenger operating unit 134 sets testees having a difference between the personality/aptitude test information and the DNA aptitude test information as subjects, extracts testees having a complementary difference between the personality/aptitude test information and the DNA aptitude test information among the subjects, matches the testees by opening a chat room for conversation between the extracted testees, and operates counselling and education.

For example, as illustrated in FIG. 8, the second messenger operating unit 134 may store, in database, personality/aptitude test information and DNA aptitude test information for testees 1, 2, 3, and 4 for whom there is a difference between personality/aptitude test information and DNA aptitude test information as illustrated in FIG. 8 and extract testees having a complementary difference between personality/aptitude test information and DNA aptitude test information for the subjects. For example, if, for testee 1 grouped to have complementary relationship 1, personality/aptitude test information a is content related to 'athlete,' and DNA aptitude test information b is content related to 'literature,' then, for testee 4 having a complementary relationship with testee 1, personality/aptitude test information b may be content related to 'literature,' and DNA aptitude test information a may be content related to 'athlete.' In other words, the personality/aptitude test information for testee 1 is similar in content to the DNA aptitude test information for testee 4, and the DNA aptitude test information for testee 1 is similar in content to the personality/aptitude test information for testee 4. The testees for whom the personality/aptitude test information and the DNA aptitude test information are complementary to each other may be extracted and grouped. For groups A and B thusly generated, a chat room for conversation between the testees may be opened to provide a chance of introduction. As necessary, a counselor may intervene to proceed with counseling and education with the testees. Such a solution may be provided.

The personality/aptitude variation trend information providing unit 140 creates a database of the personality/aptitude test information, DNA aptitude test information, and results of analysis on the solution information through the solution providing unit 130 and, upon a future personality/aptitude test on the testee, analyze a personality/aptitude variation trend and feed back information thereabout. In other words, after providing a solution for the testee, the personality/aptitude variation trend information providing unit 140 may grasp and analyze variations or alterations in the result of personality/aptitude test and provide another solution, feed back a new solution according to the changed personality/aptitude test result, thereby increasing the customization, precision, efficiency, and effectiveness for the personality/aptitude test solution.

According to an embodiment, there is provided a system for a genetic customized aptitude test which continuously provides more enhanced aptitude information by integrating epigenetic and environmental information and testee's personality/aptitude test results based on individual genetic information. The system may provide testees interested in career (occupation), education, re-education, and marriage with solutions and test results fitted for their genotypes, helping in their decision making.

## Claims

1. A system for providing a solution through fusion of a DNA aptitude test and a personality/aptitude test, the system comprising:
a personality/aptitude test information providing unit providing personality/aptitude test information through a personality/aptitude test on a testee;
a DNA aptitude test information providing unit analyzing the testee's genetic predisposition based on a result of a genetic test on the testee and providing DNA aptitude test information; and
a solution providing unit comparing the personality/aptitude test information with the DNA aptitude test information, analyzing a genetic predisposition and an environmental factor resulting from a result of the personality/aptitude test information, and providing a solution necessary for a career path and education of the testee based on a result of the analysis and the DNA aptitude test information and the personality/aptitude test information.

2. The system of claim 1, wherein the personality/aptitude test information providing unit performs the personality/aptitude test on the testee through at least one scheme of an online scheme and an offline scheme and provides the result of the personality/aptitude test on the testee.

3. The system of claim 1, wherein the DNA aptitude test information providing unit includes a genetic predisposition information providing unit determining the testee's genotype using a DNA marker having a specific genetic predisposition and providing genetic predisposition information for the testee according to a result of the determination; and a genetic aptitude information providing unit providing a score for the genetic aptitude information for the testee based on the genetic predisposition information provided through the genetic predisposition providing unit and a pre-established genetic aptitude information database.

4. The system of claim 1, wherein the solution providing unit includes a first solution providing unit comparing the personality/aptitude test information and the DNA aptitude test information for the testee, if the personality/aptitude test information is not different from the DNA aptitude test information, analyzing whether the result of the personality/aptitude test information comes from the genetic predisposition or the environmental factor, if the result of the personality/aptitude test information is analyzed as coming from the genetic predisposition, providing an environmental solution for enhancing the testee's personality/aptitude by enhancing the environmental factor, and if the result of the personality/aptitude test information is analyzed as coming from the environmental factor, providing a genetic solution for enhancing the testee's personality/aptitude by enhancing the genetic predisposition; and a second solution providing unit comparing the personality/aptitude test information and the DNA aptitude test information for the testee and, if the personality/aptitude test information is determined to be different from the DNA aptitude test information, providing a solution for enhancing the testee's personality/aptitude based on the personality/aptitude test information.

5. The system of claim 4, further comprising a personality/aptitude variation trend information providing unit creating a database for the personality/aptitude test information, the DNA aptitude test information, and a result of analysis on solution information through the solution providing unit and providing information about a personality/aptitude variation trend when the testee's personality/aptitude is retested.

6. The system of claim 4, wherein the solution providing unit further includes a first messenger operating unit measuring a similarity between the personality/aptitude test information and the DNA aptitude test information for testees, extracting testees having a preset reference similarity or more, opening a chat room for conversation between the extracted testees, matching the testees, and operating counseling and education.

7. The system of claim 4, wherein the solution providing unit further includes a second messenger operating unit setting testees having a difference between the personality/aptitude test information and the DNA aptitude test information as subjects, extracting testees among the subjects for which the personality/aptitude test information and the DNA aptitude test information are complementarily different from each other, opening a chat room for conversation between the extracted testees, matching the testees, and operating counseling and education.

8. The system of claim 1, wherein the personality/aptitude test includes at least one test item of intelligence, mathematical computational ability, positive thinking, sleep pattern, subjective euphoria, life satisfaction, neuroticism, extroverted personality, education level, information processing speed, mathematical ability, verbal memory, ability of learning from failure, altruism, athletic ability, muscle composition, injury risk, core body temperature stability during exercise, lactate threshold reaction for aerobic exercise, and exercise heart rate for regular exercise training.
